(19) Europäisches Patentamt
European Patent Office
Office européen des brevets

(11) **EP 2 870 499 B1**

(12) **EUROPEAN PATENT SPECIFICATION**

(45) Date of publication and mention
of the grant of the patent:
**11.03.2020 Bulletin 2020/11**

(21) Application number: **13813619.7**

(22) Date of filing: **02.07.2013**

(51) Int Cl.:
*B01L 9/00* *(2006.01)*        *G01N 21/64* *(2006.01)*
*G02B 21/26* *(2006.01)*        *G02B 21/34* *(2006.01)*
*G01N 33/569* *(2006.01)*        *G01N 33/543* *(2006.01)*

(86) International application number:
**PCT/US2013/049112**

(87) International publication number:
**WO 2014/008282 (09.01.2014 Gazette 2014/02)**

(54) **DIAGNOSTIC APPARATUS**

DIAGNOSEVORRICHTUNG

APPAREIL DE DIAGNOSTIC

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **03.07.2012 US 201261667691 P
27.06.2013 US 201313928741**

(43) Date of publication of application:
**13.05.2015 Bulletin 2015/20**

(73) Proprietor: **Advanced Animal Diagnostics, Inc.
Morrisville, NC 27560 (US)**

(72) Inventors:
• **BRESOLIN, Stefano**
  **Garner, North Carolina 27529 (US)**
• **CALDERWOOD, David A.**
  **Chapel Hill, North Carolina 27517 (US)**
• **HEINECK, Tobias M.**
  **Durham, North Carolina 27713 (US)**
• **NEWCOMB, David**
  **Morrisville, North Carolina 27560 (US)**
• **PAUL, Chris**
  **Hillsborough, North Carolina 27278 (US)**
• **POLLARD, Jasper N.**
  **Durham, North Carolina 27712 (US)**
• **RODRIGUEZ, Rodolfo R.**
  **Cary, North Carolina 27513 (US)**
• **YOUNG, Demetris**
  **Durham, North Carolina 27704 (US)**

(74) Representative: **Barker Brettell LLP
100 Hagley Road
Edgbaston
Birmingham B16 8QQ (GB)**

(56) References cited:
WO-A2-2004/094977        WO-A2-2008/002563
US-A- 4 440 301        US-A- 4 946 266
US-A1- 2003 127 609        US-A1- 2004 101 912
US-A1- 2007 242 349        US-A1- 2008 088 918
US-A1- 2008 259 566        US-A1- 2009 116 101
US-A1- 2010 135 861        US-A1- 2010 328 766
US-B1- 7 589 962        US-B1- 7 861 768

EP 2 870 499 B1

## Description

## Field of the Invention

The present invention concerns diagnostic apparatus, particularly apparatus useful for detecting white blood cells or analytes in bodily fluids of production animals (for example, bovine mastitis in cattle from milk).

## Background of the Invention

Mastitis is the inflammation of the mammary gland caused by microorganisms that invade one or more quadrants of the bovine udder, multiply, and produce toxins that are harmful to the mammary gland. Economic loss to mastitis in the United States is estimated to be over 2 billion dollars. This is approximately 10% of the total value of farm milk sales, and about two-thirds of this loss is due to reduced milk production in subclinically infected cows.

In subclinical mastitis, there may be no visible signs of the disease, and diagnosis of subclinical mastitis may be performed by a somatic cell count (SCC) of the milk. The SCC is the number of leukocytes or white blood cells per volume of milk and is also used as an index of milk quality. It has also been recognized that there are multiple types of leukocytes, each with its own significance. In milk from a healthy animal, the predominant cell types are lymphocytes, followed by much lesser numbers of neutrophils and macrophages. The percentages of each kind of cell rise and fall as part of the immune response to infection. Those percentages, "the differential milk leukocyte count", represent the unique immune status of an individual quarter udder, at a specific point in time for better diagnosis of subclinical mastitis.

One method for detecting the differential milk leukocyte count is using flow-cytometry, which is an expensive, sophisticated tool typically only found in top research laboratories and generally not practical for the farmer. Another method for detecting the differential milk leukocyte count is the "manual milk differential smear" (MMDS), which is a difficult and time consuming procedure, and is subject to great variability, even when performed by highly trained laboratory technologists. Both flow-cytometry and MMDS present practical difficulties for field research or a barn environment.

U.S. Patent Application Publication No. 2009/0233329 to Rodriguez discloses a wedge micro fluidic slide chamber for detecting mastitis or other diseases from a body fluid of a mammal, such as from cow's milk. While manual and automated procedures for carrying out disease detection with the aid of such a sample cartridge are described, again there is not described a system and apparatus useful for implementing such procedures in a field or barn environment.

US2009/116101 discloses a light-tight enclosure system for housing an automated microscope.

US2008/088918 discloses a microscope mounted into a computer, such as e.g., a desktop, workstation, or laptop. The microscope mounted into the computer has a light source component, a microscope component, and a light analysis component. The light source component is connected to the microscope component, which in turn is connected to the light analysis component.

US7861768 discloses a heat sink having an embedded heat pipe and fins attached to opposite sides of a base plate having a heat spreader component made of a diamond copper composite is disclosed. In various embodiments, the diamond copper composite heat spreader contains channels for receiving a main heat pipe as well as auxiliary heat pipes.

US2007/242349 discloses a system for reading microscope slides in an automated fashion.

US7589962 discloses an apparatus for cooling a heat generating component located within a portable computer system enclosure. In one embodiment a flat heat pipe is attached to the bottom surface of the portable computer keyboard support plate. The flat heat pipe is thermally coupled to one or more heat generating components housed within the computer system enclosure.

US2008/259566 discloses ways for cooling components contained in enclosures that reject 500 or more Watts employing two phase passive heat transfer devices including Loop Heat Pipes and devices referred to as LHPLs.

US2010/135861 discloses a slide holder comprising a rectangular frame having two side walls facing each other, a first end wall facing a second end wall, the second end wall having a latch pivotally attached thereto, the latch capable of being pivoted in such a manner as to enable locking and unlocking of the slide holder in a slide holder carrier. The slide holder carrier has a first end wall, a first side wall, a second end wall, a second side wall, and a shaft running from the first end wall of the slide holder carrier to the second end wall of the slide holder carrier. At least one slide holder is rotatably mounted on the shaft.

WO2008/002563 discloses a heating apparatus comprising a support base and a microplate having a first surface and an opposing second surface. The microplate is positioned adjacent the support base and comprises a plurality of wells formed in the first surface thereof. Each of the plurality of wells is sized to receive an assay therein. A sapphire crystalline transparent window is positioned adjacent the microplate opposing the support base. A heating device heats the transparent window in response to a control system.

## Summary of the Invention

The present claimed invention is directed to an automated microscope apparatus, as defined in claim 1. This apparatus comprises (a) an outer housing; and (b) a microscope assembly in said housing. The microscope assembly comprises: a support frame; a subframe; a plurality of vibration isolators connecting said support frame to said subframe; an XYZ stage connected to said subframe; an optical stage connected to said subframe; and an XYZ drive assembly interconnecting said XYZ stage to said subframe. A baseplate serves as the subframe for both the optical stage and the XYZ drive assembly, with the XYZ drive assembly and the optical stage each independently being mounted to the subframe, and wherein the baseplate is in turn mounted through vibration dampening mounts to the support frame. A sample cartridge can be removably inserted into or engaged by the XYZ stage.

In one embodiment, the automated microscope apparatus, comprises: an outer housing having an external wall; optionally but preferably an internal wall in said housing, and configured to form a first compartment and a separate second compartment in said outer housing; a microscope assembly in said housing, preferably in said first compartment; and a microprocessor in said housing, preferably in said second compartment; and optionally but preferably a heat sink mounted on said housing external wall, preferably adjacent said second compartment, with said microprocessor thermally coupled to said heat sink and operatively associated with said microscope assembly.

In the claimed invention, the microscope assembly is required to comprise: a support frame; a subframe; a plurality of vibration isolators connecting said support frame to said subframe; an XYZ stage connected to said subframe; and an optical stage connected to said subframe. An XYZ drive assembly interconnecting said XYZ stage to said subframe is included.

In some embodiments, the microprocessor is included as a passively cooled microprocessor assembly, comprising: a heat sink having a front surface and back surface; a circuit board having a front surface and back surface, with said microprocessor mounted on said circuit board front surface; a thermal coupler positioned between said microprocessor and said heat sink back surface, said thermal coupler fixed to and in thermal contact with said heat sink back surface; a clamp connected to said thermal coupler and configured to clamp said microprocessor to said thermal coupler, thereby placing said microprocessor, said thermal coupler, and said heat sink in thermal contact with one another.

In some embodiments, the XYZ stage is for securing a sample cartridge in the automated microscope having X, Y, and Z planes of movement, the sample cartridge having an end portion, a pair of generally parallel opposing side edge portions, and a locking edge portion formed thereon. The XYZ stage comprises a base member having a planar stage surface portion; a pair of generally parallel oppositely facing guide members on said planar stage surface and configured for slideably receiving said cartridge therebetween; and a locking member on said planar stage surface portion and positioned to press against the sample cartridge locking edge portion when said sample cartridge is inserted between said guide members, so that pressure is exerted by said lock member through said sample cartridge against at least one of said guide members, whereby the cartridge is removably locked in place on the XYZ stage in the Z plane.

Described but not claimed is an automated system for detecting a disorder in a subject, comprising: an XYZ stage configured to secure a sample cartridge; said sample cartridge comprising at least one chamber, said at least one chamber containing a biological sample collected from a subject; an imaging system operatively associated with said XYZ stage and configured to image selected cells in said sample, said selected cells including at least neutrophils; an autofocusing system operatively associated with said imaging system and said XYZ stage and configured to focus said imaging system on said at least one chamber; a processor running a software program or other suitable means for generating a count of at least neutrophils in said sample as an aid to detecting a disorder in said subject. Optionally, where the cartridge contains multiple chambers, the system may include a controller configured to optionally repeat at least said imaging for at least one additional chamber on said cartridge, as discussed further below.

Described but not claimed is a method of automatically focusing a microscope on a specimen by capturing an image from each of a plurality of focal planes in or on said specimen, calculating a focus score for each of said images, selecting the focal plane corresponding to the image having the best focus score, and then repositioning said specimen relative to said microscope so that said microscope is focused on said selected focal plane, characterized by including a plurality of exogenous targets in or on said specimen.

Described but not claimed is an automated microscope comprising a specimen support stage, an objective lens, a camera, at least one drive assembly operatively associated with said support stage and/or said objective lens, all of which may be as described herein, and further characterized by a controller operatively associated with said at least one drive assembly for carrying out an autofocus method as described herein.

The foregoing and other objects and aspects of the present invention are described in greater detail below.

**Brief Description of the Drawings**

**Figure 1** is a partial schematic diagram of an apparatus.
**Figure 2** is a perspective view of an apparatus of the present invention, with a sample cartridge to be inserted and touch screen user interface for input of information and display of results.
**Figure 3** is a schematic diagram of an apparatus of the present invention, showing vibration damping components and chamber separation.

**Figure 4** is a cut-away perspective view of the apparatus of Figure 2.

**Figure 5** is a side sectional view of an optical stage of the apparatus of Figure 2, showing the light source, objective lens, filters, dichroic mirror and camera.

**Figure 6** is a perspective view of a microscope assembly and passively cooled microprocessor assembly of the apparatus of Figure 2 with the cover removed and support frames removed.

**Figure 7** is a perspective view of a microscope assembly of the apparatus of Figure 2, with the support frame removed, showing the XYZ drive.

**Figure 8** is a perspective view of the mount, vibration dampers, and support frame of a microscope assembly of Figure 2, upon which the optical stage of Figure 5 is to be mounted.

**Figure 9** is a perspective view of a passively cooled microprocessor assembly of the apparatus of Figure 2.

**Figure 10** is an exploded view of the microprocessor assembly of Figure 9.

**Figure 11** is a perspective view of an XYZ stage of the apparatus of Figure 2, as configured for retaining a pair of sample cartridges.

**Figure 12** is a top plan view of the XYZ stage of Figure 11.

**Figure 13** is a side view of the XYZ stage of Figure 11.

**Figure 14** is a perspective view of the XYZ stage of Figure 11, showing a first sample cartridge seated in place, and a second sample cartridge to be inserted.

**Figure 15** is a perspective view of an alternate XYZ stage for an apparatus of Figure 2, in which a single sample cartridge is to be inserted.

**Figure 16** is a perspective view of the XYZ stage of Figure 15, with a sample cartridge inserted.

**Figure 17** is a schematic flow chart of a first mode of operation of an apparatus of Figure 2 for detecting mastitis in cattle.

**Figure 18** illustrates the display of a user interface of an apparatus of Figure 2 during homing of the optical stage;

**Figure 19** illustrates the display of a user interface of an apparatus of Figure 2 for input of animal data or information, particularly the identity of the animal from which the sample(s) are collected;

**Figure 20** illustrates the display of a user interface of an apparatus of Figure 2 for input of animal data or information, particularly the type of sample collected, and the number of chambers in the sample cartridge for which sample imaging and analysis is to be carried out;

**Figure 21** illustrates the display of a user interface of an apparatus of Figure 2 after homing and/or information entry is completed and when the apparatus is ready to receive the sample cartridge.

**Figure 22** illustrates the display of a user interface of an apparatus of Figure 2 during image acquisition and analysis of one of the four separate chambers of a sample cartridge.

**Figure 23** illustrates the display of a user interface of an apparatus of Figure 2 after image acquisition and differential leukocyte analysis has been completed. Note that one of the four quarters is indicated as "positive" for mastitis.

## Detailed Description of Illustrative Embodiments

The present invention will now be described more fully hereinafter, in which embodiments of the invention are shown. This invention may, however, be embodied in different forms and should not be construed as limited to the embodiments set forth herein. Rather, these embodiments are provided so that this disclosure will be thorough and complete, and will fully convey the scope of the invention, which is defined by the claims, to those skilled in the art. In the drawings, like numbers refer to like elements throughout. Thicknesses and dimensions of some components may be exaggerated for clarity.

Unless otherwise defined, all terms (including technical and scientific terms) used herein have the same meaning as commonly understood by one of ordinary skill in the art to which this invention belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

The terminology used herein is for the purpose of describing particular embodiments only and is not intended to be limiting of the invention. As used herein, the singular forms "a", "an" and "the" are intended to include the plural forms as well, unless the context clearly indicates otherwise. It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof. As used herein the expression "and/or" includes any and all combinations of one or more of the associated listed items.

In addition, spatially relative terms, such as "under", "below", "lower", "over", "upper" and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "under" or "beneath" other elements or features would then be oriented "over" the other elements or features. Thus, the exemplary term "under" can encompass both an orientation of over and under. The device may be otherwise oriented (rotated 90 degrees or at other orientations) and the spatially relative descriptors used herein interpreted accordingly.

Well-known functions or constructions may not be described in detail for brevity and/or clarity.

"Subject" as used herein includes both human and animal subjects for veterinary purposes, as well as plants for agricultural purposes. Examples of animal subjects include, but are not limited to, mammalian subjects such as dog, cat, cow, sheep, goat, llama, alpaca, camel, horse, pig, chicken, and turkey subjects.

Dairy animals such as cows, goats, sheep, buffalo, and camel, for the production of milk are particularly preferred for some embodiments of the invention.

"Milk" as used herein generally refers to mammalian milk of any species (e.g., cow, goat, human, etc.). The milk is typically raw milk, and is typically raw milk produced by dairy cattle. In some embodiments "milk" includes colostrum; in other embodiments "milk" refers to milk intended for human consumption after the production of colostrum has ceased. The milk may optionally be diluted (typically with an aqueous diluent such as distilled water, saline solution, or buffer solution).

"Colostrum" as used herein is a form of milk produced by mammals in the first few days after birth, that may be higher in antibodies (for imparting passive immunity to offspring).

"Secretions" as used herein is a form of milk produced by mammals just prior to giving birth. Such secretions are sometimes also referred to as "colostrum" but in the present application "secretions" refers to the type of milk produced prior to the subject giving birth, while colostrum refers to the type of milk produced just after the subject giving birth.

"Sample cartridge" or "diagnostic cartridge" as used herein may be any suitable cartridge for containing a cell sample, including but are not limited to cartridges suitable for differential leukocyte analysis as described In R. Rodriguez and C. Galanaugh, US Patent Application Publication No. 2009/0233329 (published September 17, 2009), and optionally incorporating the modifications or features discussed further below. In general, and as illustrated further below, such as cartridge includes at least one (e.g., two, four) sample chambers (e.g., a microfluidic chamber), which chamber or chambers may contain suitable cell or leukocyte observation colorants, stains, or reagents (e.g., reagents suitable for visualizing the cells under epifluorescent microscopy). The sample chambers are preferably aligned with one another on the cartridge (that is, on substantially the same Z plane as one another on the cartridge). In a preferred embodiment, each chamber contains reagents for separately and distinctly imaging or detecting neutrophils (or "polymorphonuclear leukocytes" (PMN)), lymphocytes, and macrophages, for differential leukocyte count diagnosis of infections such as bovine mastitis, in accordance with procedures known in the art, or which will be apparent to those skilled in the art based upon the instant disclosure, as discussed further below.

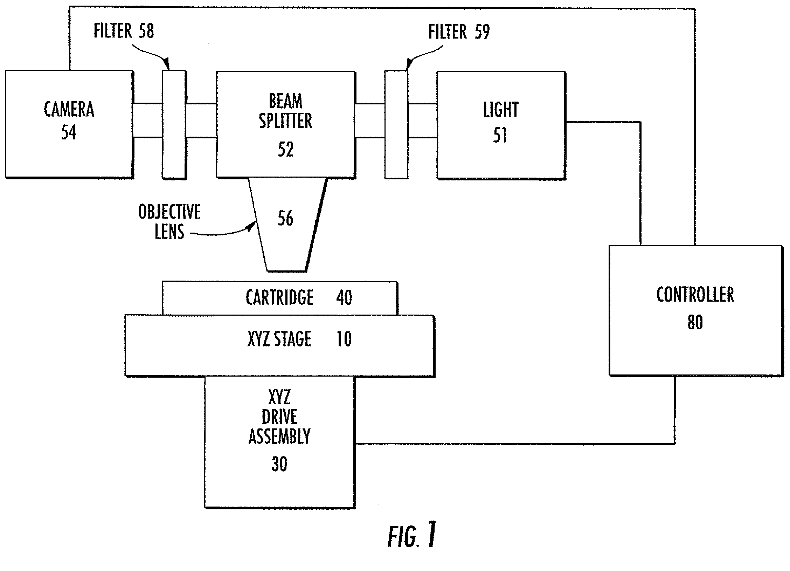

A partial schematic diagram of an apparatus is given as an overview in **Figure 1.** The apparatus comprises an XYZ stage **(10)** mounted on an XYZ drive assembly **(30)**. A sample cartridge **(40)** is removably inserted into or engaged by the XYZ stage. The optical components for carrying out epifluorescent microscopy include a light or light source **(51)**, a beam splitter **(52)**, a camera **(54)**, and an objective lens **(56)**, all configured so that light from the source is directed onto the sample cartridge, and light emitted or fluoresced from the sample cartridge is directed to the camera. Filters **(58, 59)** are provided between the camera and beam splitter, and between the light source and beam splitter, so that the appropriate wavelengths of light are directed onto the sample cartridge, and the appropriate wavelengths of light are directed onto the camera. All components including the camera, light, and XYZ drive assembly, are controlled by any suitable controller **(80),** which may comprise a computer or microprocessor with associated memory units, power, and additional control boards (not always shown) such as an XYZ controller board.

Individual components of the methods and apparatus described herein may be as known in the art, or variations thereof that will be apparent to those skilled in the art based on the instant disclosure and prior automated microscopy apparatus such as described in US Patents Nos. 4,998,284 to Bacus; 5,548,661 to Price; 5,790,710 to Price; 6,381,058 to Ramm; 6,929,953 to Wardlaw; 6,927,903 to Stuckey; 8,000,511 to Perz; 8,045,165 to Wardlaw; 8,081,303 to Levine; or US Patent Application No. 2001/0041347 to Sammak.

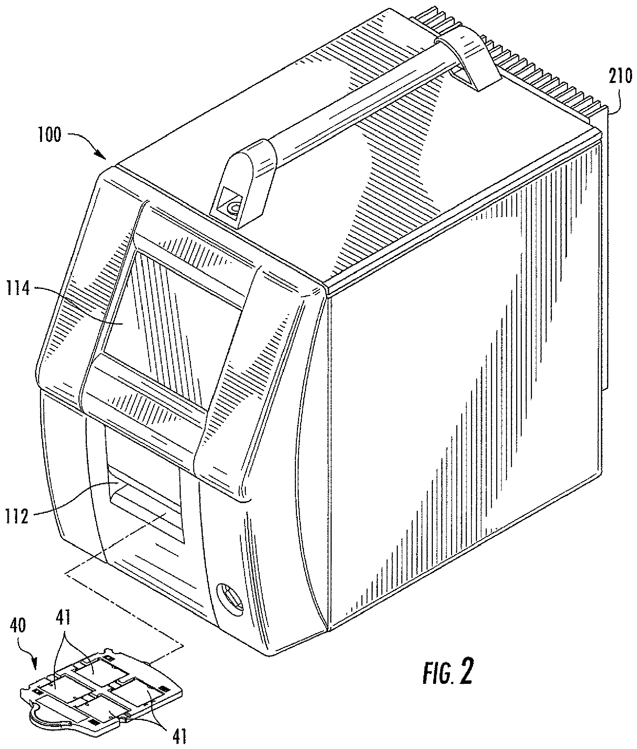

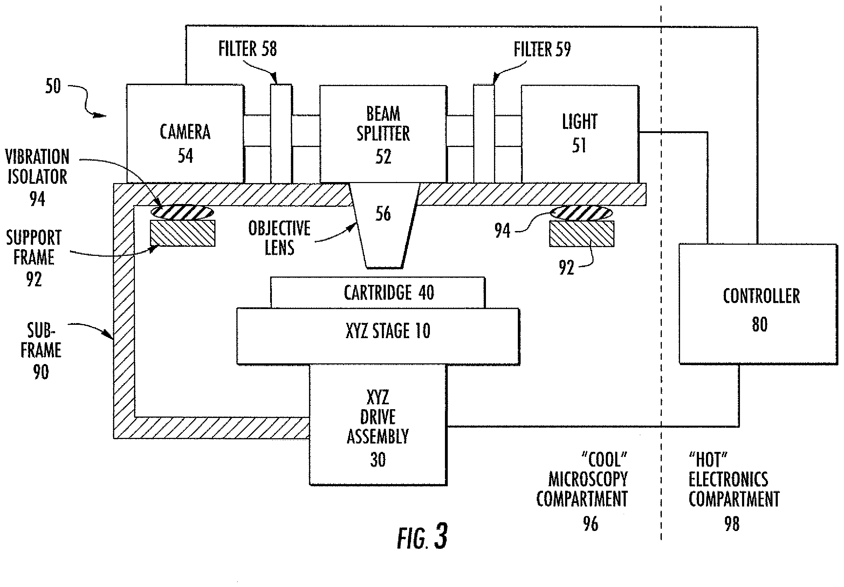

**Figure 2** is a perspective view of an apparatus **(100)** of the present invention, as constructed for portability and use in a dusty or otherwise harsh environment such as a barn or farm, or out-of-doors where animals to be diagnosed are found. All components of Figure 1 above (and Figure 3 below) are contained within the housing, except for the sample cartridge, which is removably inserted through a suitable opening **(112)** in the housing. A touch screen display **(114)** on the front of the device (e.g., an ESTECOM 6.5 inch intelligent panel LCD display/monitor) is provided to both display results and control the apparatus through its operational steps, as discussed further below.

**Figure 3** is a schematic diagram of an apparatus of the present invention similar to Figure 1 above. In addition to the components shown in Figure 1, additional features are now shown. The optical components **(50)** are shown as mounted on a subframe **(90)**, which subframe is in turn mounted on a support frame **(92)** through vibration isolators **(94)**. In addition, the microscopy components are shown as being contained within a separate, relatively cool, compartment **(96)** from the controller, which is in a relatively hot or warm compartment **(98)** (as compared to the microscopy compartment). The apparatus of Figure 2 above incorporates these additional features, as discussed further below.

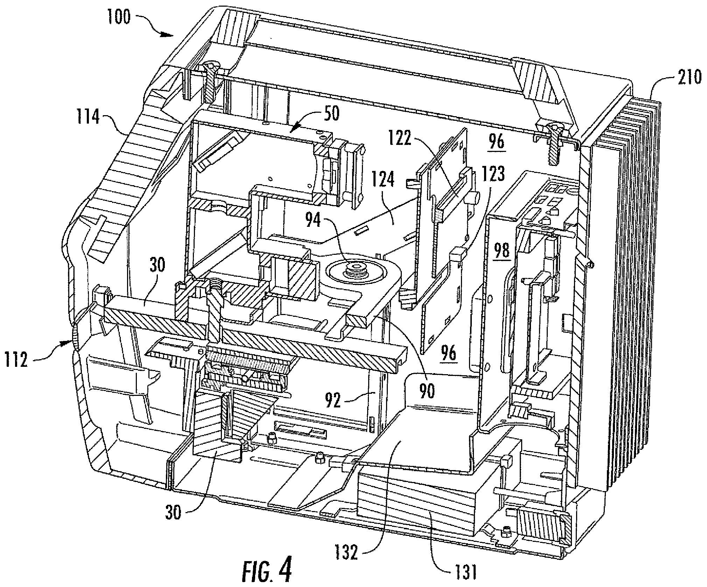

A partial cut-away perspective view of the apparatus of Figure 2 is given in **Figure 4.** A baseplate **(90)** serves a subframe for both the optical stage **(50)** and the XYZ drive assembly **(30),** which baseplate is in turn mounted through vibration dampening mounts **(94)** to the support frame **(92).** Any suitable active or passive vibration mount may be used, such as polymeric vibration mounts (*e.g.,* those available from Stock Drive Products/Sterling Instruments, or any other suitable source).

An XYZ controller board **(122)** and a power distribution board **(123)** are conveniently located on a support bracket (**124**), which support bracket is mounted on the support frame **(92),** to facilitate assembly and testing of the microscopy compartment elements before they are placed into the housing, though numerous other configurations will be apparent to those skilled in the art.

A suitable power supply **(131)** (*e.g.,* a fanless power supply such as MEAN WELL USP-350-12 350W power supply) is positioned in the bottom of the unit and covered by a shield or cable tray **(132)** (cables not shown for clarity) to prevent tangling of cables associated with the XYZ drive assembly, image sensor, and/or light, though numerous other configurations will be apparent, including location of the power supply external to the main housing.

A heat sink **(210)** is mounted on the back of the apparatus to cool the electronics compartment, as discussed further below.

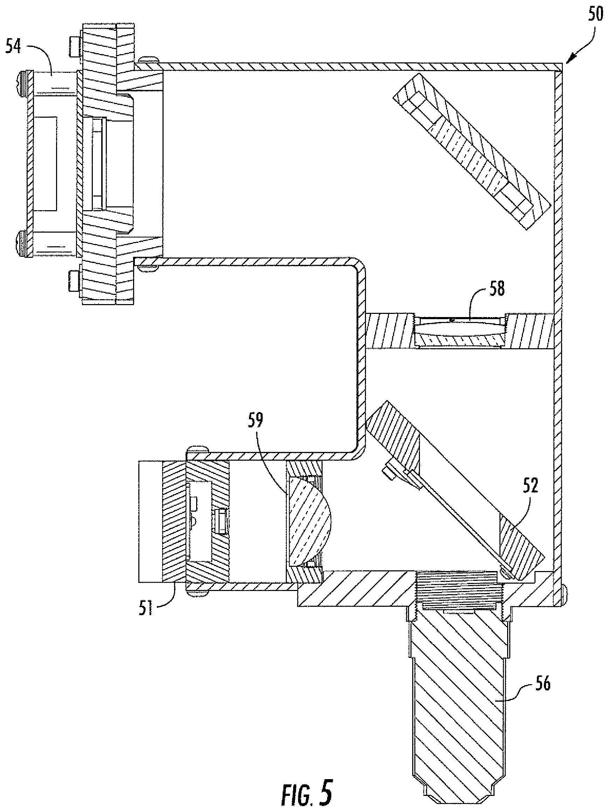

**Figure 5** is a side sectional view of an optical stage of the apparatus of Figure 2, showing the light source, objective lens, filters including emission filters and excitation filters, dichroic mirror and image sensor (sometimes also referred to as "camera" herein), all contained within or connected to a common housing. Any suitable image sensor may be used, including CMOS image sensors, CCD image sensors, and hybrids thereof, typically 1 or 2 megapixel up to 10 or 20 megapixel, or more in resolution (*e.g.,* a 5.0 megapixel OPTIC ANGLE image sensor). Any suitable light source may be used, including LED light (*e.g.* a CREE LED). Any suitable objective lens may be used, such as a 5x to 50x or 100x magnification objective lens (*e.g.,* a NIKON MRL 00102 10x objective lens). In some embodiments, the light source is a 480 nm light source or LED; the emission filter is a dual pass filter with the center wavelength of 530 nm and 700 nm; the excitation filter has a center wave length of 470 nm, the dichroic mirror reflects 470 nm light and transmits light greater than 490 nm).

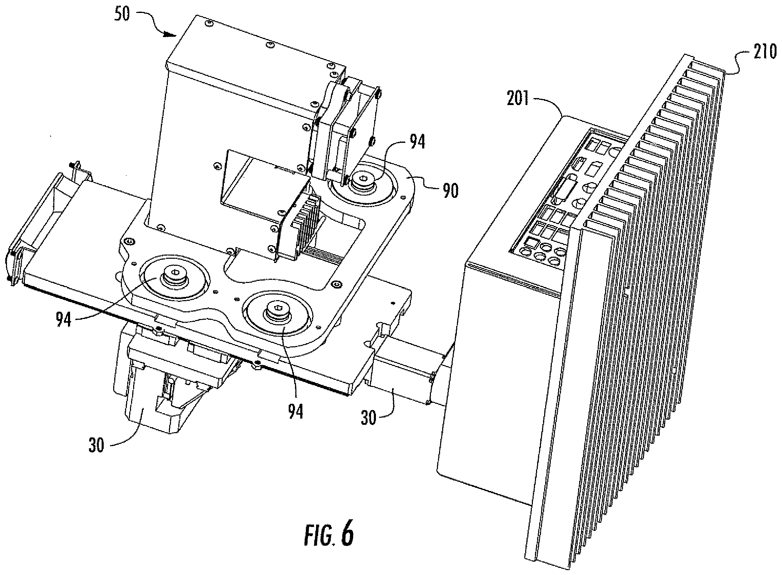

The relationship of the major components of the microscopy compartment to the separate electronics compartment is shown in **Figure 6,** which is a perspective view of a microscope assembly and passively cooled microprocessor assembly of the apparatus of Figure 2 with the cover removed and support frame removed, showing the housing **(201)** surrounding the microprocessor board contained within the passively cooled electronics compartment. A solid state hard drive (not shown) may be conveniently mounted on the external surface of the electronics compartment housing to provide memory and storage, if desired, though again numerous other configurations will be readily apparent.

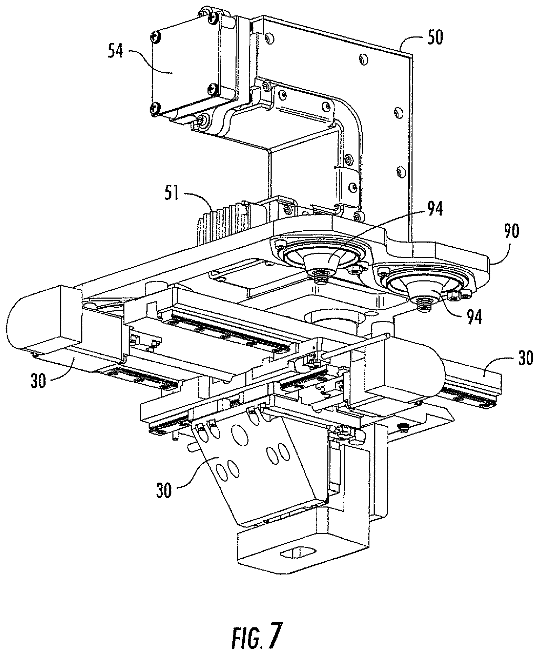

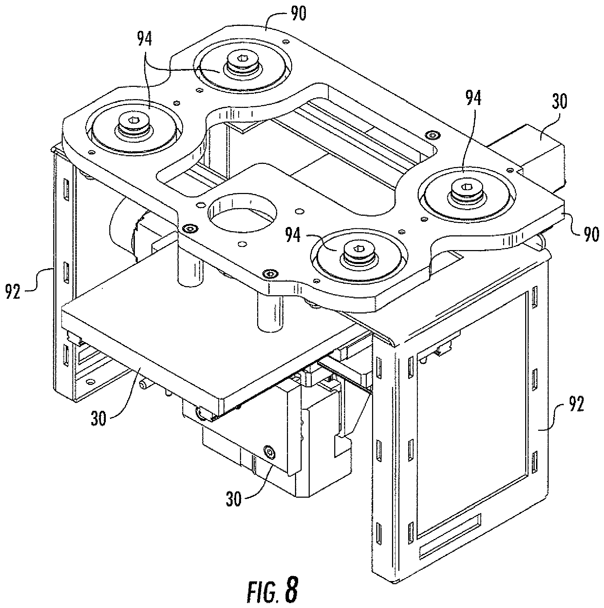

The various components of the microscopy compartment are further illustrated in **Figures 7-8.** Figure 7 is a lower perspective view of a microscope assembly of the apparatus of Figure 2, showing the XYZ drive assembly mounted to the base plate (subframe), the optical stage mounted to the subframe, and the vibration isolation bushings, but with the support frame removed. Similarly, **Figure 8** is an upper perspective view of the base plate (subfame), XYZ drive assembly mounted on the base plate, mount, support frame upon which the base plate (subframe) is mounted through the vibration isolation bushings, but now with the optical stage removed.

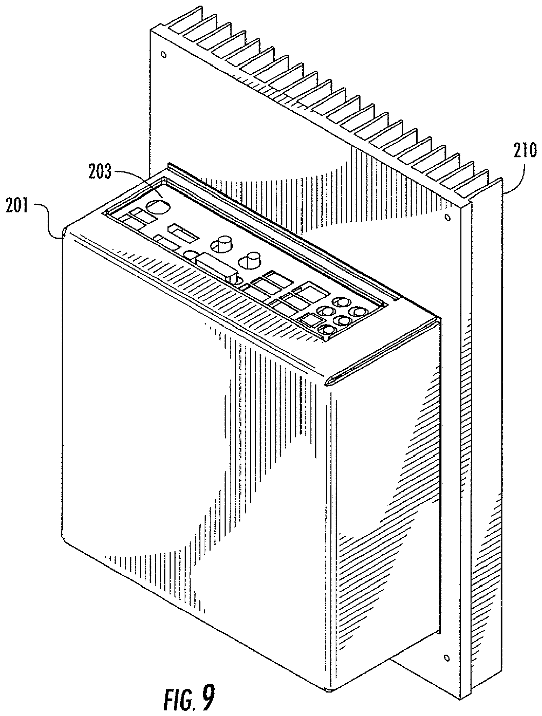

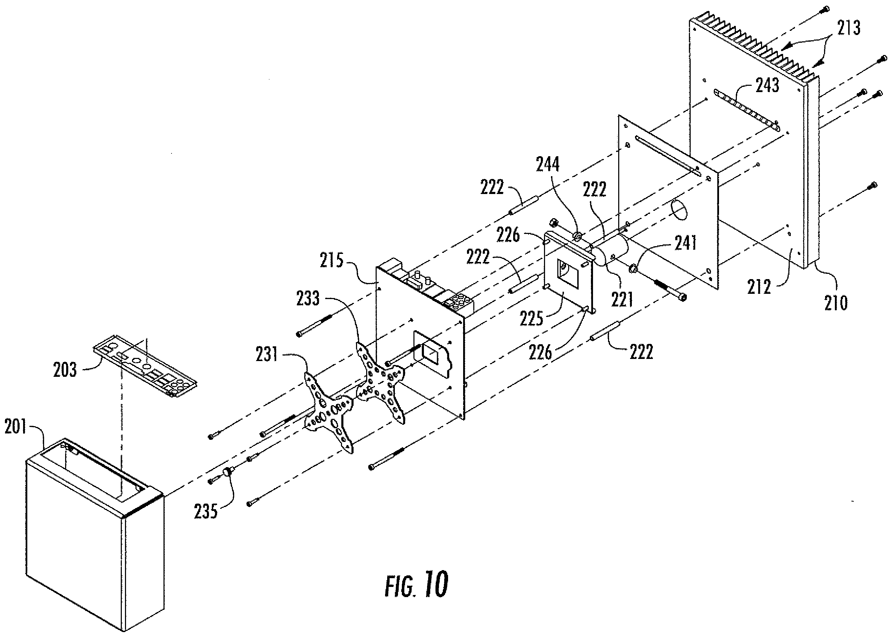

**Figure 9** is a perspective view of a passively cooled electronics compartment of the apparatus of Figure 2, showing the electronics compartment housing (in which the microprocessor assembly is contained) mounted on the heat sink. An exploded view of this electronics compartment and microprocessor assembly is shown in **Figure 10.** A mother board (*e.g.,* a ZOTAC H67ITX-CE motherboard) is provided that carries a suitable microprocessor. Suitable microprocessors will generally be those having a thermal design power (or "TDP", sometimes also called "thermal design point") of at least 40, 50, or 60 Watts, up to 120, 140, or 160 Watts, or more. Suitable examples include, but are not limited to, Intel i7, Intel i5, and Intel i3 microprocessors.

As will be seen from Figures 9-10, a passively cooled microprocessor assembly includes a heat sink **(210)** having a front surface and back surface **(212),** the heat sink having cooling posts, fins or other suitable projections **(213)** formed on the front surface. A circuit board **(215)** or "mother board" having a front surface and back surface is included, with a microprocessor mounted on the circuit board front surface. A thermal coupler **(221)** (*e.g.,* a copper slug or member; a heat pipe; etc.) is positioned between the microprocessor and said heat sink back surface, with the thermal coupler fixed to and in thermal contact with said heat sink back surface. A plurality of legs **(222)** are mounted on the heat sink back surface, with the circuit board mounted on the legs, and with the circuit board front surface spaced from and facing said heat sink back surface.

An anchor plate **(225)** is positioned around the microprocessor between the heat sink back surface and the circuit board front surface, with the anchor plate connected to the thermal coupler. A plurality of posts **(226)** are connected to the anchor plate and project through the circuit board, with a primary plate **(231)** connected to the posts opposite the anchor plate with the circuit board therebetween. A secondary plate **(233)** is slideably received on the plurality of posts and contacts said circuit board back surface. A screw **(235)** is threaded through the primary plate and contacts the secondary plate, so that tightening of the screw pushes the secondary plate against the circuit board back surface and clamps said microprocessor to said heat sink (optionally but preferably with a thermal grease sandwiched in between), thereby fixing the microprocessor, the thermal coupler, and the heat sink in thermal contact with one another. A housing (201) (*e.g.,* a metal or aluminum) with an associated bezel (203) is provided around the assembly to form an electronics compartment (98) in the device separate from the microscopy compartment, as noted above. There is preferably included at least one thermal isolator (241) formed from a relatively thermally nonconductive material (*e.g.*, an organic polymer), with the thermal coupler and the anchor plate are connected to one another through the at least one thermal isolator.

A ventilation opening (243) such as an elongated slot may optionally be formed in the heat sink to further facilitate cooling of the electronics chamber. Such an opening or port is preferably configured to inhibit or slow the progression of liquid or solid particles from outside the apparatus entering into the electronics chamber, such as by configuring the slot at a downward angle.

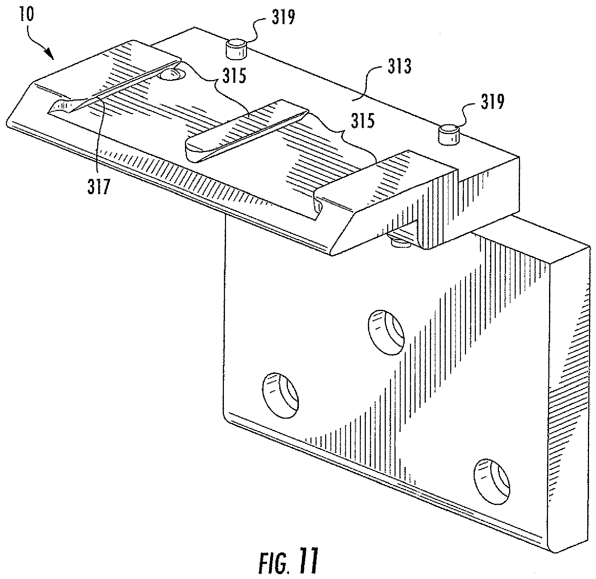

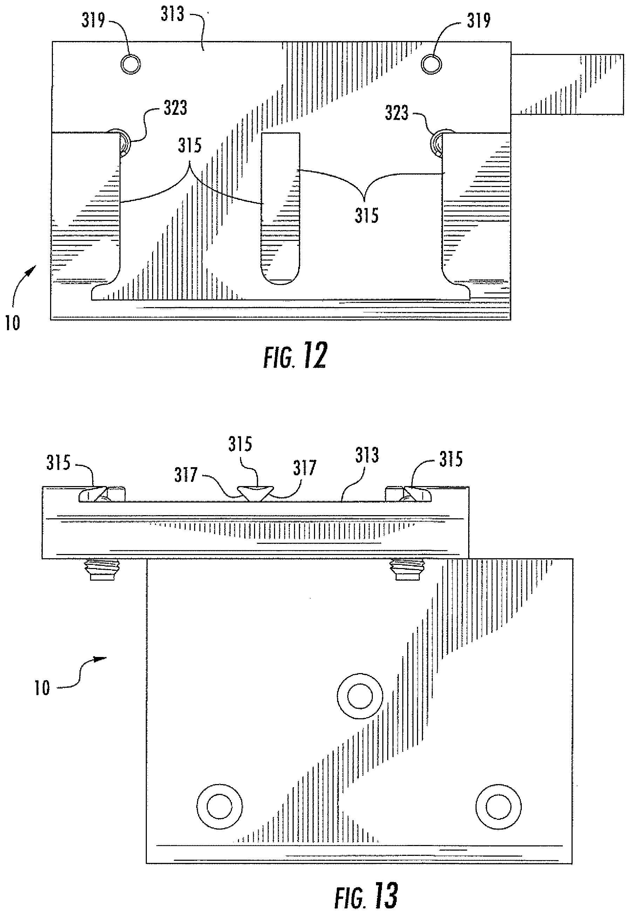

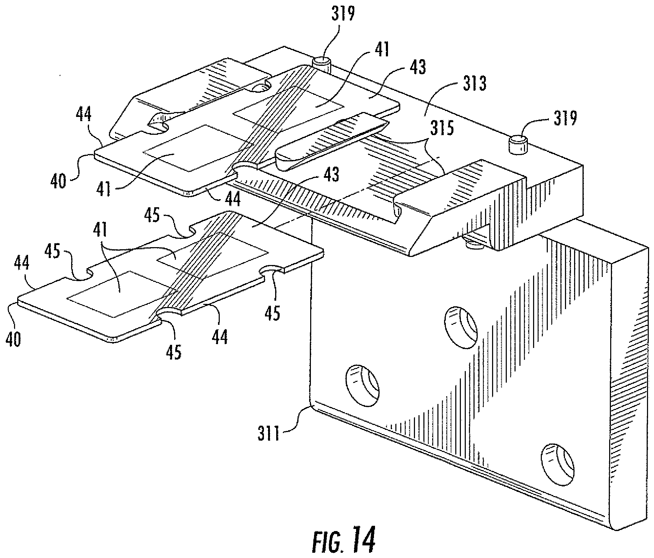

**Figures 11 to 14** illustrate a first embodiment of an XYZ stage (10) of the apparatus of Figure 2, as configured for retaining a pair of sample cartridges (40). As illustrated, each sample cartridge contains a pair of separate chambers (41), and the sample cartridges are reversibly insertable into the XYZ stage. One or both of the chambers may optionally contain exogenous targets to facilitate autofocus, as described below.

As shown in Figures 11 to 14, such a stage is configured to receive a sample cartridge having an end portion (43), a pair of generally parallel opposing side edge portions (44), and a locking edge portion formed (45) thereon, with each of said side edge portions having an upper corner portion, and with said locking edge portion positioned at an angle in relation to both said side portions and said front portion. The XYZ stage itself comprises a base member (311) having a planar stage surface portion (313), and a pair of generally parallel oppositely facing guide members (315) on said planar stage surface, each of said guide members having an inwardly angled edge portion (317) configured for contacting one of the cartridge side edge upper corner portions when the sample cartridge is inserted therebetween. A terminal block member (319) is provided on the planar stage surface portion and positioned to contact the sample cartridge end portion when the sample cartridge is inserted between said guide members. A locking member (323) (*e.g.,* a spring-loaded ball detent) is included on the planar stage surface portion and positioned to press against the sample cartridge locking edge portion when the sample cartridge is inserted between the guide members and in contact with said terminal block, so that pressure is exerted by said lock member through said sample cartridge against both said terminal block and one of said guide members, whereby the cartridge is removably locked in place on the XYZ stage in at least the Z plane of movement, preferably all three of the X, Y and Z planes of movement, and still more preferably with the cartridge secured with reference to, or with respect to, the X, Y, and Z axes of rotation as well.

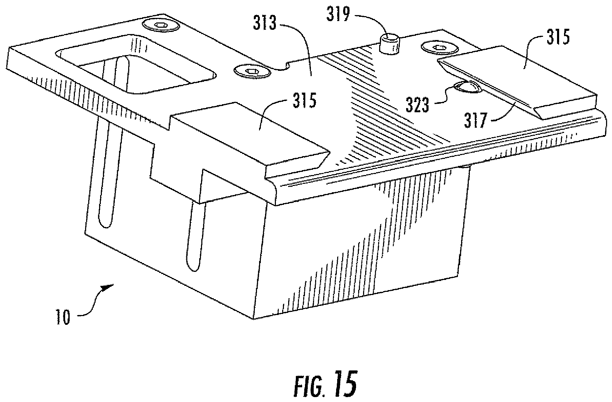

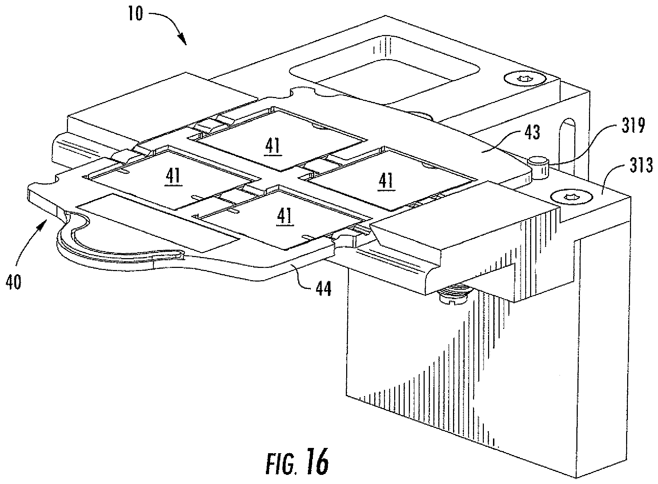

**Figure 15 to 16** illustrate a second embodiment of an XYZ stage (10) of an apparatus of Figure 2, as configured for retaining a single sample cartridge (40). Like components as compared found in Figures 11 to 14 are assigned like numbers. As illustrated in Figures 15-16, the sample cartridge contains four separate chambers (41) (sometimes also referred to as "quadrants" or "quads"), each of which may (for example) be used to contain a milk, colostrum or secretions sample from a separate one of each of the four teats of a cow's udder. One, some, or all of the chambers may optionally contain exogenous targets to facilitate autofocus, as described below. As illustrated, the sample cartridge is nonreversible, or is configured so that it may be inserted into the XYZ stage in a single orientation only. When each teat of origin of a milk sample deposited within each chamber is identified or recorded, this facilitates identification of an infected teat or gland for subsequent treatment, and/or aids in identifying the severity or extent of infection of a particular cow.

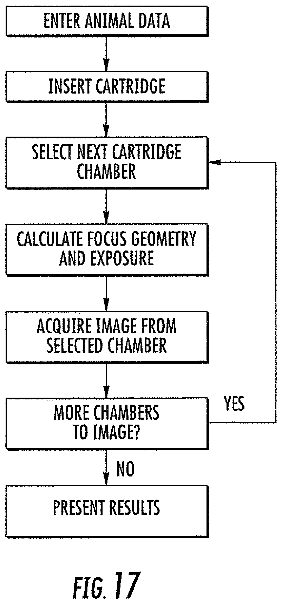

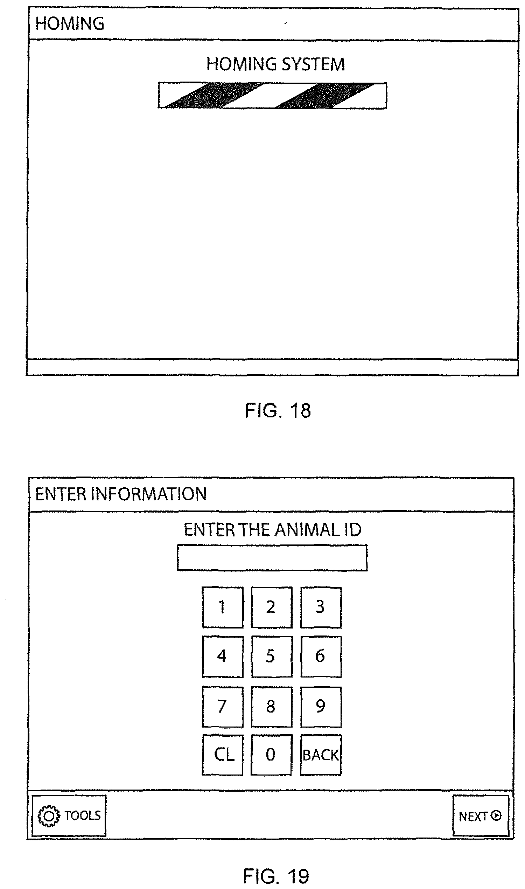

**Figures 17** illustrate a mode of operating a device as described above, with **Figures 18-23** illustrating the images displayed on (*i.e.*, "screen shots" from) the user interface or "touch screen" of the apparatus of Figure 2 described above. All components including the XYZ drive assembly, the light, the camera or imaging device, and the touch screen, may be operatively associated with and controlled by the controller or microprocessor as discussed above, programmed in a suitable language such as MICROSOFT C#.

Upon activating the system, the XYZ stage can be "homed" in accordance with known techniques, such as with electromechanical sensors, during which time a "homing" message such as shown in **Figure 18** may be displayed on the display screen.

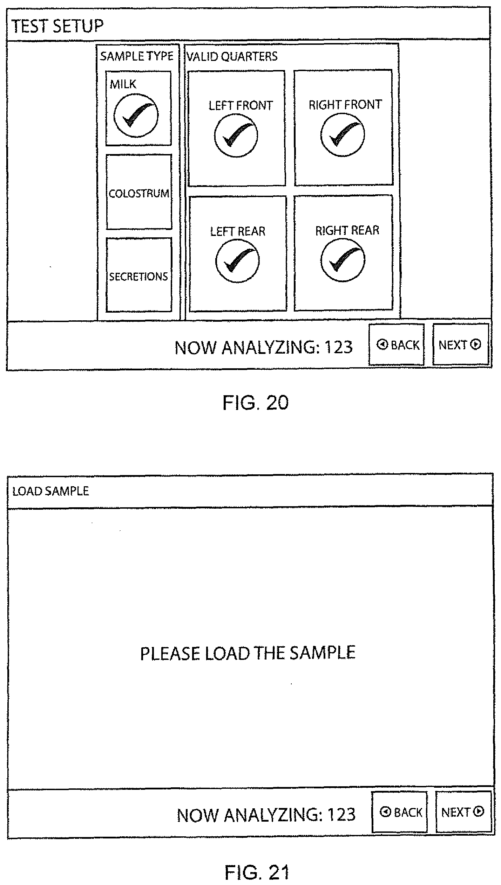

As shown in Figure 17, following the process may begin (before or after "homing") by entering animal data, such as an animal identification or "ID" through a display interface such as shown in **Figure 19.** Before or after animal identification is entered, the type of sample to be screened may be selected (*e.g.,* milk, colostrum, secretions), and/or the number of separate chambers to be analyzed can be entered (which, in the case of a cow, can correspond to the quadrant of the mammary gland, and/or the specific teat, from which the sample is collected), such as through a suitable display and data entry screen such as shown in **Figure 20.** Elimination of one or more chambers from the analysis procedure may advantageously reduce the overall time of the test.

The sample cartridge may be inserted (before or after the entry of the animal data), optionally as prompted through the display of a "load sample" or "load cartridge" message such as given in **Figure 21.** If desired, access to the cartridge carrier may be secured through a manually operated door, or an automated door controlled by the controller to open, and close, at the appropriate time in the operating cycle.

After the sample cartridge is inserted, the microscope is autofocused on the first sample chamber (as shown in Figure 17) and imaging (including identification and counting of cells of interest) is carried out on the first sample chamber. Autofocusing may be carried out by any suitable technique, including but not limited to those described in US Patents Nos. 8,014,583; 7,141,773; 5,790,710; 5,647,025; 5,483,055; and 4,810,869, and variations thereof that will be apparent to those skilled in the art. In some embodiments, autofocusing is carried out prior to acquisition of an image of the specimen or sample through the camera, typically through calculating a focus score. The focus score can be calculated by any suitable technique, including but not limited to those described in F. Groen et al., A comparison of different focus functions for use in autofocus algorithms, Cytometry 6, 81-91 (1985). Difference from the background, given a uniform background, can be calculated a number of ways, including but not limited to differences in contrast, gradient, and variance.

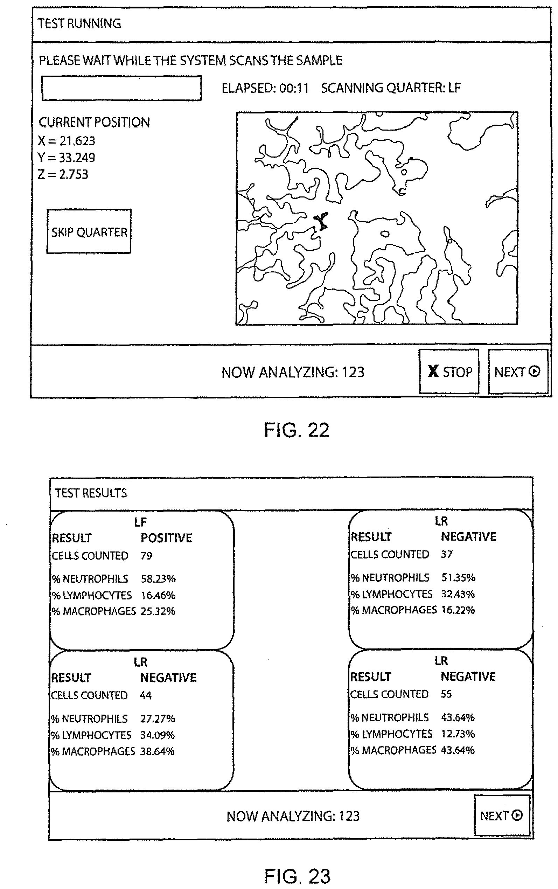

A display such as shown in **Figure 22** may optionally be provided during imaging, giving information such as the microscope image and the position (XY, and optionally Z) being scanned or imaged. Once imaging of the first chamber is completed, the optical stage is positioned by the controller over the next sample chamber to be imaged, again autofocused thereon as described above, and again imaged as described above. This process is repeated until all sample chambers have been imaged. In the alternative, an input signal can be provided to the controller to omit sampling of a particular chamber, such as through the touch screen **115,** for example by selecting individual "valid quarters" through the "left front", "right front", "left rear", and "right rear" buttons of the screen shown in Figure 20, and/or by a "skip quarter" button as shown in Figure 22.

Identification and counting of cells can be carried out in accordance with known techniques or variations thereof that will be apparent to those skilled in the art. *See, e.g.,* A. Katz, Image Analysis and Supervised Learning in the Automated Differentiation of White Blood Cells from Microscopic Images, Master's Thesis (Royal Melbourne Institute of Technology 2000); *see also* US Patent No. 7,991,213 to Tafas and US Patent Application Nos. 2004/0085443 to Kallioniemi; 2011/0182490 to Hoyt; 2011/0255753 to Levenson; and 2011/0255745 to Hodder.

Determination of infection can be carried out from cell counts and identities in accordance with known techniques or variations thereof that will be apparent to those skilled in the art, such as by total leukocyte count or differential leukocyte count. *See, e.g.,* Rodriguez and Galanaugh, *supra;* H. Tvedten et al., Automated differential leukocyte count in horses, cattle, and cats using the Technicon H-1E hematology system, Vet. Clin Pathol. 25, 14-22 (1996); G. Leitner et al., Milk leucocyte population patterns in bovine udder infection of different aetiology, J. Vet. Med. B. Infect Dis. Vet. Public Health 47, 581-89 (2000); H. Dosogne et al., Differential Leukocyte Count Method for Bovine Low Somatic Cell Count Milk, J. Dairy Sci. 86, 828-834 (2003); M. Albenzio et al., Differential Leukocyte Count for Ewe Milk with Low and High Somatic Cell Count, J. Dairy Research 78, 43-48 (2011).

Results of imaging, identification, counting and analysis can be printed, stored on a suitable memory, and/or displayed on a final image screen such as that shown in **Figure 23.**

**Exogeneous targets.**

General considerations for selecting the exogeneous target are as follows: The exogenous target should be visible by the particular optical system in use. This will depend on the magnification, excitation wavelength, size of field of view, etc. This will influence decisions on which size, shape, emission wavlengths, etc. of the texture. In addition, the exogenous target should be distinguishable from the target objects. Preferably, the exogeneous target resides at sustantially the same (or a known distance from) the focal plane of the target objects (*e.g.,* be mixed with a biological sample suspected of containing cells to be imaged and/or counted, and/or placed in the same chamber as will contain a biological sample comprising cells to be imaged and/or counted). The exogeneous target should be of a size, shape, and number so as to not substantially obscure the view of the intended target objects, such as cells to be imaged and/or counted. And, the exogenous target should provide sufficient contrast with an empty field of view so as to provide an adequate focal peak and allow for reliable, reasonably rapid, and/or robust focusing.

The exogeneous targets may be formed of any suitable material, including organic polymers, inorganic materials (including crystalline materials, amorphous materials, metals, etc.) and composites thereof.

The exogeneous targets may be contained loosely within the chamber, fixed to one wall of the chamber, or surface to be imaged (e.g., by adhesive, by electrostatic, hydrophilic, or hydrophobic interaction, covalent bond directly or through a linking group, etc.), and/or formed on one wall of the chamber (*e.g.,* by molding, etching, painting, silk-screening, lithography, etc.).

The exogeneous targets may be opaque or transparent. When transparent the targets may be "tinted" so as to transmit light therethrough at a predetermined wavelength (for example, so that they appear red, green, blue, yellow, etc., to a human observer).

The exogeneous targets may be regular or irregular in shape (for example, cylinders, spheres, cubes, pyramids, prisms, cones, rods, etc.). In some embodiments, the targets have an average diameter of from 0.1, 0.5 or 1 micrometers up to 2, 5, or 10 micrometers.

The number of exogenous targets is not critical, but in some embodiments the speed of the autofocus process can be increased by increasing, at least to a point, the number of exogenous targets in the chamber so that the targets are readily located in the automated microscope. Where a plurality of targets are included in the sample chamber (*e.g.*, 2, 4, 6, 8 or 10 targets, up to 100, 200, 400, 600 or 800 exogenous targets, or more), in some embodiments that plurality preferably consists of or consists essentially of targets having substantially the same size, shape, and optical characteristics.

In some embodiments, the targets are beads, such as fluorescent microbeads. Such microbeads are commonly available and used for calibrating flow cytometers or fluorescent microscopes (*see, e.g.,* US Patents Nos. 4,698,262; 4,714,682; and 4,868,126).

The targets are preferably optically distinguishable from cells to be counted (and hence would not be useful as calibration standards for the particular cells to be counted and/or imaged by the methods described herein). Optically distinguishable may be achieved by any suitable technique, such as by utilizing targets of a different and distinguishable shape from the cells to be counted, by utilizing targets that emit, transmit, and/or reflect light at a different wavelength from the cells to be counted when under the same illumination conditions, and combinations thereof.

Selected aspects of the present invention are explained in greater detail in the following non-limiting Examples.

## EXAMPLE 1

### Exogenous Target-Assisted Autofocus

An example is carried out by addition of microscopic fluorescent beads to a sample to be imaged, in combination with an automated microscope including an XYZ stage under the control of a computer. A sufficient concentration of such beads will ensure that there is a very high probability of having beads within any given field of view, thereby ensuring that there is sufficient texture for the autofocus algorithm.

In general, when an automated microscope focuses, a typical approach is a sequence as follows:

1. Move to some Z location.
2. Mathematically process the digital image to obtain a "score" of the image that represents, in relative terms, whether the field of view is in focus.
3. Repeat steps 1 and 2 until a peak is found in the focus graph. This peak will represent the position at which that field of view is in best focus.

## EXAMPLE 2

### Sample or Surface Interpolation

By including exogeneous focal targets at a plurality of separate locations in the sample to be imaged, or on the sample carrier surface to be imaged (so long as cells/analytes to be imaged and focus particles are in the same image plane or "Z stack"), the surface or sample can be interpolated by inclusion of a suitable interpolation program, routine or subroutine within the autofocus subroutine, to thereby facilitate imaging of the sample, or speed imaging of the sample.

Such interpolation can be carried out by any suitable algorithm or method, including but not limited to the planar best fit method, the weighted least squares fit method, and the quadratic fit method. Such procedures are known and described in, for example, I. Coope, "Circle fitting by linear and nonlinear least squares". Journal of Optimization Theory and Applications 76 (2): 381 (1993); Ake Bjorck, Numerical Methods for Least Squares Problems, Society for Industrial and Applied Mathematics (April 1996); etc.

The planar best fit method is illustrated by the equation:

$$z = Ax + By + C$$

Method 1 involves the average of x, y and z points; Method 2: Least Squares Linear Regression; and Method 3: Weighted Least Squares Regression. Data: x, y, and z focus points collected outside the viewing/imaging sample area. At least 3 data points are required.

The quadratic fit method is illustrated by the equation:

$$z = Ax^2 + By^2 + Cxy + Dx + Ey + F.$$

The method involves a second order quadratic surface. Data: x, y, and z focus points are collected somewhere outside the viewing/imaging/sample area. At least six data points are required.

When the cells to be imaged are collected and imaged within the same enclosed chamber, the exogeneous targets may be simply included in the chamber. When cells to be imaged are captured by antibodies bound to a carrier surface, the sample is collected on a surface that carries antibodies that bind the cells. Antibodies may be covalently or non-covalently coupled to the surface by any suitable technique as is known in the art.

To carry out interpolation, it is preferable that the exogenous targets be in or on the chamber, or on the (generally planar, but not always perfectly planar) surface supporting the specimen or sample to be imaged, at a plurality of locations. While in some embodiments 3 locations will be sufficient, in other embodiments 4, 5, or 6 or more locations are desired. The locations may be separate and discrete from one another (that is, without exogenous target deposited therebetween) or may be contiguous (that is, with exogenous target therebetween). Spacing between the locations will in general be determined by factors such as magnification and the size of the sample to be imaged (particularly in the XY dimensions), but in some embodiments the locations will be spaced apart at least 10, 20, or 30 percent of the average width of the sample support surface or chamber to be imaged. Such spacing may be achieved by depositing the exogenous targets at discrete locations around the region where the antibodies are deposited, by depositing the exogenous targets at discrete locations among the region where the antibodies are deposited, by depositing exogenous targets on at least a major portion, or all of, the support surface or chamber to be imaged, etc.

**Claims**

1. An automated microscope apparatus, comprising:

   (a) an outer housing; and
   (b) a microscope assembly in said housing;

   said microscope assembly comprising:

   a support frame;
   a subframe;
   a plurality of vibration isolators connecting said support frame to said subframe;
   an XYZ stage connected to said subframe;
   an optical stage connected to said subframe; and
   an XYZ drive assembly interconnecting said XYZ stage to said subframe;
   wherein a baseplate serves as the subframe for both the optical stage and the XYZ drive assembly, with the XYZ drive assembly and the optical stage each independently being mounted to the subframe, and wherein the baseplate is in turn mounted through vibration dampening mounts to the support frame;
   and wherein a sample cartridge can be removably inserted into or engaged by the XYZ stage.

2. The microscope apparatus of claim 1, wherein the optical stage comprises optical components for carrying out epifluorescent microscopy and includes a light or light source, a beam splitter, a camera, and an objective lens, all configured so that light from the source is directed onto the sample cartridge, and light emitted or fluoresced from the sample cartridge is directed to the camera, and wherein filters are provided, between the camera and beam splitter and between the light source and beam splitter.

3. The microscope apparatus of claim 1, wherein said XYZ stage is configured to receive a sample cartridge having an end portion, a pair of generally parallel opposing side edge portions, and a locking edge portion formed thereon;
   said XYZ stage comprising:

   a base member having a planar stage surface portion;
   a pair of generally parallel oppositely facing guide members on said planar stage surface and configured for slideably receiving said cartridge therebetween; and
   a locking member on said planar stage surface portion and positioned to press against the sample cartridge locking edge portion when said sample cartridge is inserted between said guide members, so that pressure is exerted by said lock member through said sample cartridge against at least one of said guide members, whereby the cartridge is removably locked in place on the XYZ stage in the Z plane.

4. The microscope apparatus of claim 3, wherein said sample cartridge includes at least one sample chamber, which chamber or chambers may contain suitable cell or leukocyte observation colorants, stains, or reagents.

5. The microscope apparatus of claim 4, wherein said sample cartridge includes two or more sample chambers.

6. The microscope apparatus of claim 5, wherein the sample chambers are aligned with one another on the cartridge, that is, on substantially the same Z plane as one another on the cartridge.

7. The microscope apparatus of claim 5 or claim 6, wherein each chamber contains reagents for separately and distinctly imaging or detecting neutrophils (or "polymorphonuclear leukocytes"), lymphocytes, and macrophages, for differential leukocyte count diagnosis of infections.

8. The microscope apparatus of claim 7, wherein the reagents are for separately and distinctly imaging or detecting neutrophils (or "polymorphonuclear leukocytes"), lymphocytes, and macrophages, for differential leukocyte count diagnosis of bovine mastitis.

9. The microscope apparatus of any one of claims 4-8, wherein each chamber is a microfluidic chamber.

10. The microscope apparatus of claim 3,
    wherein the apparatus is configured for retaining a pair of sample cartridges, wherein the sample cartridges are reversibly insertable into the XYZ stage;

and wherein each of said side edge portions has an upper corner portion, and wherein said locking edge portion is positioned at an angle in relation to both said side portions and said edge portion, and wherein a terminal block member is provided on the planar stage surface portion and is positioned to contact the sample cartridge end portion when the sample cartridge is inserted between said guide members, and wherein each of said guide members has an inwardly angled edge portion configured for contacting one of the cartridge side edge upper corner portions when the sample cartridge is inserted therebetween, and wherein said locking member is positioned to press against the sample cartridge locking edge portion when the sample cartridge is inserted between the guide members and in contact with said terminal block.

11. The microscope apparatus of claim 3, wherein the locking member is a spring-loaded ball detent.

12. The automated microscope of any one of claims 1-11, said passively cooled microprocessor assembly comprising:

> a heat sink having a front surface and back surface;
> a circuit board having a front surface and back surface,
> a microprocessor mounted on said circuit board front surface;
> a thermal coupler positioned between said microprocessor and said heat sink back surface, said thermal coupler fixed to and in thermal contact with said heat sink back surface; and
> a clamp connected to said thermal coupler and configured to clamp said microprocessor to said thermal coupler, thereby placing said microprocessor, said thermal coupler, and said heat sink in thermal contact with one another.

13. The automated microscope of claim 12, wherein said thermal coupler comprises a copper slug or heat pipe.

14. The automated microscope of claim 13, further comprising:

> a) at least one interconnecting member formed from a thermally nonconductive material, wherein said thermal coupler and said clamp are connected to one another through said at least one interconnecting member; or
> b) a plurality of legs connected to said heat sink back surface, said circuit board mounted on said legs, with said circuit board front surface spaced from and facing said heat sink back surface.

15. The automated microscope of claim 13, wherein:

> a) said clamp comprises a rigid frame, a movable clamping member, and a screw or lever interconnecting said frame and clamping member, with said rigid frame connected to said thermal coupler, and said clamping member configured to clamp said microprocessor to said thermal coupler; or
> b) said heat sink has cooling projections fins on said heat sink front surface; or
> c) said microprocessor has a thermal design power rating of at least 40 Watts.

**Patentansprüche**

1. Automatisierte Mikroskopvorrichtung, umfassend:

> (a) ein Außengehäuse und
> (b) eine Mikroskopanordnung in dem Gehäuse,

wobei die Mikroskopanordnung Folgendes umfasst:

> einen Tragrahmen,
> einen Hilfsrahmen,
> eine Vielzahl den Tragrahmen mit dem Hilfsrahmen verbindender Schwingungsisolatoren,
> ein mit dem Hilfsrahmen verbundenes XYZ-Gestell,
> ein mit dem Hilfsrahmen verbundenes optisches Gestell und
> eine das XYZ-Gestell mit dem Hilfsrahmen verbindende XYZ-Antriebsanordnung,
> wobei eine Grundplatte als Hilfsrahmen für sowohl das optische Gestell als auch die XYZ-Antriebsanordnung dient, wobei die XYZ-Antriebsanordnung und das optische Gestell jeweils unabhängig am Hilfsrahmen befestigt sind und wobei die Grundplatte ihrerseits durch schwingungsdämpfende Halterungen am Hilfsrahmen befestigt ist,
> und wobei eine Probenkassette lösbar in das XYZ-Gestell eingesetzt oder von diesem gegriffen werden kann.

2. Mikroskopvorrichtung nach Anspruch 1, wobei das optische Gestell optische Komponenten zum Durchführen von Auflichtfluoreszenzmikroskopie umfasst und ein Licht oder eine Lichtquelle, einen Strahlteiler, eine Kamera und ein Objektiv beinhaltet, die alle so konfiguriert sind, dass Licht aus der Quelle auf die Probenkassette gelenkt wird und von der Probenkassette emittiertes oder fluoresziertes Licht zur Kamera gelenkt wird, und wobei zwischen der Kamera und dem Strahlteiler sowie zwischen der Lichtquelle und dem Strahlteiler Filter bereitgestellt sind.

**3.** Mikroskopvorrichtung nach Anspruch 1, wobei das XYZ-Gestell dafür konfiguriert ist, eine Probenkassette aufzunehmen, die einen Endabschnitt, ein Paar allgemein paralleler gegenüberliegender Seitenrandabschnitte und einen darauf gebildeten Sperrrandabschnitt aufweist, wobei das XYZ-Gestell Folgendes umfasst:

> ein Sockelelement mit einem ebenen Gestellflächenabschnitt,
> ein Paar allgemein paralleler, einander gegenüberliegender Führungselemente auf der ebenen Gestellfläche, die dafür konfiguriert sind, die Kassette gleitend zwischen sich aufzunehmen, und
> ein Sperrelement auf dem ebenen Gestellflächenabschnitt, das so positioniert ist, dass es gegen den Sperrrandabschnitt der Probenkassette drückt, wenn die Probenkassette zwischen den Führungselementen eingesetzt ist, so dass Druck von dem Sperrelement durch die Probenkassette gegen mindestens eines der Führungselemente ausgeübt wird, wodurch die Kassette lösbar auf dem XYZ-Gestell in der Z-Ebene arretiert ist.

**4.** Mikroskopvorrichtung nach Anspruch 3, wobei die Probenkassette mindestens eine Probenkammer beinhaltet, wobei die Kammer oder Kammern für Zell- oder Leukozytenuntersuchung geeignete Farbstoffe, Anfärbungen oder Reagenzien enthalten können.

**5.** Mikroskopvorrichtung nach Anspruch 4, wobei die Probenkassette zwei oder mehr Probenkammern beinhaltet.

**6.** Mikroskopvorrichtung nach Anspruch 5, wobei die Probenkammern auf der Kassette miteinander ausgerichtet sind, das heißt, auf der Kassette im Wesentlichen miteinander auf der gleichen Z-Ebene liegen.

**7.** Mikroskopvorrichtung nach Anspruch 5 oder Anspruch 6, wobei jede Kammer Reagenzien zum separaten und unterscheidenden Darstellen oder Erfassen von Neutrophilen (oder "polymorphkernigen Leukozyten"), Lymphozyten und Makrophagen zur mittels Differentialleukozytenbild erfolgenden Diagnose von Infektionen enthält.

**8.** Mikroskopvorrichtung nach Anspruch 7, wobei die Reagenzien zum separaten und unterscheidenden Darstellen oder Erfassen von Neutrophilen (oder "polymorphkernigen Leukozyten"), Lymphozyten und Makrophagen zur mittels Differentialleukozytenbild erfolgenden Diagnose boviner Mastitis vorgesehen sind.

**9.** Mikroskopvorrichtung nach einem der Ansprüche 4 bis 8, wobei es sich bei jeder Kammer um eine Mikrofluidikkammer handelt.

**10.** Mikroskopvorrichtung nach Anspruch 3, wobei die Vorrichtung dafür konfiguriert ist, ein Paar Probenkassetten zu halten, wobei die Probenkassetten reversibel in das XYZ-Gestell einsetzbar sind, und wobei jeder der Seitenrandabschnitte einen Oberkantenabschnitt aufweist und wobei der Sperrrandabschnitt in einem Winkel zu sowohl den Seitenabschnitten als auch dem Randabschnitt positioniert ist, und wobei auf dem ebenen Gestellflächenabschnitt ein Anschlussblockelement bereitgestellt und so positioniert ist, dass dieses den Endabschnitt der Probenkassette kontaktiert, wenn die Probenkassette zwischen den Führungselementen eingesetzt ist, und wobei jedes der Führungselemente einen nach innen geneigten Randabschnitt aufweist, der dafür konfiguriert ist, einen der Kassettenseitenrand-Oberkantenabschnitte zu kontaktieren, wenn die Probenkassette zwischen diesen eingesetzt ist, und wobei das Sperrelement so positioniert ist, dass es gegen den Sperrrandabschnitt der Probenkassette drückt, wenn die Probenkassette zwischen den Führungselementen eingesetzt und mit dem Anschlussblock in Kontakt ist.

**11.** Mikroskopvorrichtung nach Anspruch 3, wobei es sich bei dem Sperrelement um eine gefederte Kugelarretierung handelt.

**12.** Automatisiertes Mikroskop nach einem der Ansprüche 1 bis 11, wobei die passiv gekühlte Mikroprozessoranordnung Folgendes umfasst:

> eine Wärmesenke mit einer Vorderseite und einer Rückseite,
> eine Leiterplatte mit einer Vorderseite und einer Rückseite,
> einen auf der Leiterplatten-Vorderseite befestigten Mikroprozessor,
> eine zwischen dem Mikroprozessor und der Wärmesenken-Rückseite positionierte Wärmekopplungseinrichtung,
> wobei die Wärmekopplungseinrichtung an der Wärmesenken-Rückseite fixiert und mit dieser in thermischem Kontakt ist, und
> eine mit der Wärmekopplungseinrichtung verbundene Klammer, die dafür konfiguriert ist, den Mikroprozessor an die Wärmekopplungseinrichtung zu klemmen, wodurch der Mikroprozessor, die Wärmekopplungseinrichtung und die Wärmesenke in thermischen Kontakt miteinander gebracht werden.

**13.** Automatisiertes Mikroskop nach Anspruch 12, wobei die Wärmekopplungseinrichtung einen Kupferblock oder einen Röhrenkühlkörper umfasst.

**14.** Automatisiertes Mikroskop nach Anspruch 13, ferner umfassend:

a) mindestens ein aus einem nicht-wärmeleitenden Material gebildetes Verbindungsstück, wobei die Wärmekopplungseinrichtung und die Klammer durch mindestens ein Verbindungselement miteinander verbunden sind, oder
b) eine Vielzahl mit der Wärmesenken-Rückseite verbundener Beine, wobei die Leiterplatte an den Beinen befestigt ist, wobei die Leiterplatten-Vorderseite der Wärmesenken-Rückseite zugewandt und zu dieser beabstandet ist.

**15.** Automatisiertes Mikroskop nach Anspruch 13, wobei:

a) die Klammer einen starren Rahmen, ein bewegliches Klemmelement und eine Schraube oder einen Hebel umfasst, die bzw. der den Rahmen und das Klemmelement verbindet, wobei der starre Rahmen mit der Wärmekopplungseinrichtung verbunden ist und das Klemmelement dafür konfiguriert ist, den Mikroprozessor an die Wärmekopplungseinrichtung zu klemmen, oder
b) die Wärmesenke auf der Wärmesenken-Vorderseite Kühlrippenvorsprünge aufweist oder
c) der Mikroprozessor eine thermische Verlustleistung von mindestens 40 Watt aufweist.

**Revendications**

**1.** Appareil de microscopie automatisé, comprenant :

(a) un boîtier extérieur ; et
(b) un ensemble de microscope dans ledit boîtier ;

ledit ensemble de microscope comprenant :

un châssis de support ;
un sous-châssis ;
une pluralité d'isolateurs de vibrations reliant ledit châssis de support audit sous-châssis ;
une platine XYZ reliée audit sous-châssis ;
une platine optique connectée audit sous-châssis ; et
un ensemble d'entraînement XYZ interconnectant ladite platine XYZ audit sous-châssis ;
une plaque de base servant de sous-châssis à la fois pour la platine optique et l'ensemble d'entraînement XYZ, l'ensemble d'entraînement XYZ et la platine optique étant chacun indépendamment montés sur le sous-châssis, et la plaque de base étant à son tour montée, au moyen de montages d'amortissement des vibrations, sur le châssis de support ;
et une cartouche d'échantillonnage pouvant être insérée de manière amovible dans ou engagée par la platine XYZ.

**2.** Appareil de microscopie selon la revendication 1, la platine optique comprenant des composants optiques pour effectuer une microscopie à épifluorescence et comprenant une lumière ou une source de lumière, un séparateur de faisceau, une caméra et un objectif, tous configurés de sorte que la lumière provenant de la source est dirigée sur la cartouche d'échantillon, et la lumière émise ou fluorescente provenant de la cartouche d'échantillon est dirigée vers la caméra, et des filtres étant prévus entre la caméra et le séparateur de faisceau et entre la source de lumière et le séparateur de faisceau.

**3.** Appareil de microscopie selon la revendication 1, ladite platine XYZ étant configurée pour recevoir une cartouche d'échantillon ayant une partie d'extrémité, une paire de parties de bord latéral opposées généralement parallèles, et une partie de bord de verrouillage formée sur celle-ci ;
ladite platine XYZ comprenant :

un élément de base ayant une partie de surface de platine plane ;
une paire d'éléments de guidage généralement parallèles et opposés sur ladite surface plane de la platine et configurés pour recevoir de manière coulissante ladite cartouche entre eux ; et
un élément de verrouillage sur ladite partie de surface plane de la platine et positionné pour exercer une pression contre la partie de bord de verrouillage de la cartouche d'échantillon lorsque ladite cartouche d'échantillon est insérée entre lesdits éléments de guidage, de sorte que la pression est exercée par ledit élément de verrouillage à travers ladite cartouche d'échantillon contre au moins un desdits éléments de guidage, de sorte que la cartouche est verrouillée en place de manière amovible sur la platine XYZ dans le plan Z.

**4.** Appareil de microscopie selon la revendication 3, ladite cartouche d'échantillon comprenant au moins une chambre d'échantillon, laquelle chambre ou lesquelles chambres peuvent contenir des colorants, des teintures ou des réactifs approprié(e)s pour l'observation de cellules ou de leucocytes.

**5.** Appareil de microscopie selon la revendication 4, ladite cartouche d'échantillon comprenant deux ou plus de deux chambres d'échantillons.

**6.** Appareil de microscopie selon la revendication 5, les chambres d'échantillons étant alignées les unes par rapport aux autres sur la cartouche, c'est-à-dire sur sensiblement le même plan Z que les unes par rapport aux autres sur la cartouche.

**7.** Appareil de microscopie selon la revendication 5 ou la revendication 6, chaque chambre contenant des réactifs pour l'imagerie ou la détection séparée et distincte des neutrophiles (ou « leucocytes polymorphonucléaires »), des lymphocytes et des macrophages, pour le diagnostic différentiel des infections par comptage des leucocytes.

**8.** Appareil de microscopie selon la revendication 7, les réactifs étant destinés à l'imagerie ou à la détection séparée et distincte des neutrophiles (ou « leucocytes polymorphonucléaires »), des lymphocytes et des macrophages, pour le diagnostic différentiel de la mammite bovine par comptage des leucocytes.

**9.** Appareil de microscopie selon l'une quelconque des revendications 4 à 8, chaque chambre étant une chambre microfluidique.

**10.** Appareil de microscopie selon la revendication 3, l'appareil étant configuré pour retenir une paire de cartouches d'échantillon, les cartouches d'échantillon étant insérables de manière réversible dans la platine XYZ ;
et chacune desdites parties de bord latéral ayant une partie de coin supérieur, et ladite partie de bord de verrouillage étant positionnée selon un angle par rapport auxdites deux parties latérales et à ladite partie de bord, et un élément de bloc terminal étant prévu sur la partie de surface plane de la platine et étant positionné pour entrer en contact avec la partie d'extrémité de la cartouche d'échantillon lorsque la cartouche d'échantillon est insérée entre lesdits éléments de guidage, et chacun desdits éléments de guidage ayant une partie de bord inclinée vers l'intérieur configurée pour entrer en contact avec l'une des parties de coin supérieur du bord latéral de la cartouche lorsque la cartouche d'échantillon est insérée entre elles, et ledit élément de verrouillage étant positionné pour exercer une pression contre la partie de bord de verrouillage de la cartouche d'échantillon lorsque la cartouche d'échantillon est insérée entre les éléments de guidage et en contact avec ledit bloc terminal.

**11.** Appareil de microscopie selon la revendication 3, l'élément de verrouillage étant une détente à bille à ressort.

**12.** Microscope automatisé selon l'une quelconque des revendications 1 à 11, ledit ensemble microprocesseur refroidi passivement comprenant :

un dissipateur thermique ayant une surface avant et une surface arrière ;
une carte de circuit imprimé ayant une surface avant et une surface arrière,
un microprocesseur monté sur la face avant de ladite carte de circuit imprimé ;
un coupleur thermique positionné entre ledit microprocesseur et ladite surface arrière du dissipateur thermique, ledit coupleur thermique étant fixé à et en contact thermique avec ladite surface arrière du dissipateur thermique ; et
une pince connectée audit coupleur thermique et configurée pour fixer ledit microprocesseur audit coupleur thermique, plaçant ainsi ledit microprocesseur, ledit coupleur thermique et ledit dissipateur thermique en contact thermique les uns avec les autres.

**13.** Microscope automatisé selon la revendication 12, ledit coupleur thermique comprenant un tube de cuivre ou un caloduc.

**14.** Microscope automatisé selon la revendication 13, comprenant en outre :

a) au moins un élément d'interconnexion formé d'un matériau thermiquement non conducteur, ledit coupleur thermique et ladite pince étant reliés l'un à l'autre au moyen dudit au moins un élément d'interconnexion ; ou
b) une pluralité de pattes reliées à ladite surface arrière du dissipateur thermique, ladite carte de circuit imprimé étant montée sur lesdites pattes, la surface avant de la carte de circuit imprimé étant espacée de la surface arrière du dissipateur thermique et faisant face à celle-ci.

**15.** Microscope automatisé selon la revendication 13,

a) ladite pince comprenant un châssis rigide, un élément de serrage mobile, et une vis ou un levier interconnectant ledit châssis et l'élément de serrage, ledit châssis rigide étant connecté audit coupleur thermique, et ledit élément de serrage étant configuré pour serrer ledit microprocesseur sur ledit coupleur thermique ;
b) ledit dissipateur thermique étant muni d'ailettes de refroidissement en saillie sur la surface avant dudit dissipateur thermique ; ou
c) ledit microprocesseur ayant une puissance thermique nominale d'au moins 40 Watts.

FILTER 58          FILTER 59

| CAMERA 54 | | BEAM SPLITTER 52 | | LIGHT 51 |

OBJECTIVE LENS        56

CARTRIDGE    40

XYZ STAGE    10

XYZ DRIVE ASSEMBLY 30

CONTROLLER 80

*FIG. 1*

EP 2 870 499 B1

*FIG. 2*

CAMERA 54

FILTER 58

BEAM SPLITTER 52

FILTER 59

LIGHT 51

CONTROLLER 80

56

OBJECTIVE LENS

CARTRIDGE 40

XYZ STAGE 10

XYZ DRIVE ASSEMBLY 30

94

92

"COOL" MICROSCOPY COMPARTMENT 96

"HOT" ELECTRONICS COMPARTMENT 98

50

VIBRATION ISOLATOR 94

SUPPORT FRAME 92

SUB-FRAME 90

*FIG. 3*

FIG. 4

FIG. 5

FIG. 6

EP 2 870 499 B1

FIG. 7

FIG. *8*

FIG. 9

FIG. 10

FIG. 11

FIG. 12

FIG. 13

FIG. 14

FIG. 15

FIG. 16

ENTER ANIMAL DATA

↓

INSERT CARTRIDGE

↓

SELECT NEXT CARTRIDGE
CHAMBER

↓

CALCULATE FOCUS GEOMETRY
AND EXPOSURE

↓

ACQUIRE IMAGE FROM
SELECTED CHAMBER

↓

MORE CHAMBERS
TO IMAGE? —— YES

↓ NO

PRESENT RESULTS

FIG. 17

HOMING

HOMING SYSTEM

FIG. 18

ENTER INFORMATION

ENTER THE ANIMAL ID

| 1 | 2 | 3 |
| 4 | 5 | 6 |
| 7 | 8 | 9 |
| CL | 0 | BACK |

{🔧} TOOLS                                                    NEXT ⊙

FIG. 19

## TEST SETUP

SAMPLE TYPE

MILK ✓

COLOSTRUM

SECRETIONS

VALID QUARTERS

LEFT FRONT ✓

RIGHT FRONT ✓

LEFT REAR ✓

RIGHT REAR ✓

NOW ANALYZING: 123    ◁ BACK    NEXT ▷

FIG. 20

## LOAD SAMPLE

PLEASE LOAD THE SAMPLE

NOW ANALYZING: 123    ◁ BACK    NEXT ▷

FIG. 21

---

TEST RUNNING

PLEASE WAIT WHILE THE SYSTEM SCANS THE SAMPLE

ELAPSED: 00:11   SCANNING QUARTER: LF

CURRENT POSITION
X = 21.623
Y = 33.249
Z = 2.753

SKIP QUARTER

NOW ANALYZING: 123      **X** STOP   NEXT ⊙

**FIG. 22**

---

TEST RESULTS

LF
RESULT          POSITIVE
CELLS COUNTED   79

% NEUTROPHILS   58.23%
% LYMPHOCYTES   16.46%
% MACROPHAGES   25.32%

LR
RESULT          NEGATIVE
CELLS COUNTED   37

% NEUTROPHILS   51.35%
% LYMPHOCYTES   32.43%
% MACROPHAGES   16.22%

LR
RESULT          NEGATIVE
CELLS COUNTED   44

% NEUTROPHILS   27.27%
% LYMPHOCYTES   34.09%
% MACROPHAGES   38.64%

LR
RESULT          NEGATIVE
CELLS COUNTED   55

% NEUTROPHILS   43.64%
% LYMPHOCYTES   12.73%
% MACROPHAGES   43.64%

NOW ANALYZING: 123      NEXT ⊙

**FIG. 23**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090233329, Rodriguez
- US 2009116101 A
- US 2008088918 A
- US 7861768 B
- US 2007242349 A
- US 7589962 B
- US 2008259566 A
- US 2010135861 A
- WO 2008002563 A
- US 4998284 A, Bacus
- US 5548661 A, Price
- US 5790710 A, Price
- US 6381058 B, Ramm
- US 6929953 B, Wardlaw
- US 6927903 B, Stuckey
- US 8000511 B, Perz
- US 8045165 B, Wardlaw
- US 8081303 B, Levine
- US 20010041347 A, Sammak
- US 35012350 W
- US 8014583 B
- US 7141773 B
- US 5647025 A
- US 5483055 A
- US 4810869 A
- US 7991213 B, Tafas
- US 20040085443 A, Kallioniemi
- US 20110182490 A, Hoyt
- US 20110255753 A, Levenson
- US 20110255745 A, Hodder
- US 4698262 A
- US 4714682 A
- US 4868126 A

### Non-patent literature cited in the description

- **F. GROEN et al.** A comparison of different focus functions for use in autofocus algorithms. *Cytometry,* 1985, vol. 6, 81-91
- **A. KATZ.** Image Analysis and Supervised Learning in the Automated Differentiation of White Blood Cells from Microscopic Images. *Master's Thesis (Royal Melbourne Institute of Technology,* 2000
- **H. TVEDTEN et al.** Automated differential leukocyte count in horses, cattle, and cats using the Technicon H-1E hematology system. *Vet. Clin Pathol.,* 1996, vol. 25, 14-22
- **G. LEITNER et al.** Milk leucocyte population patterns in bovine udder infection of different aetiology. *J. Vet. Med. B. Infect Dis. Vet. Public Health,* 2000, vol. 47, 581-89
- **H. DOSOGNE et al.** Differential Leukocyte Count Method for Bovine Low Somatic Cell Count Milk. *J. Dairy Sci.,* 2003, vol. 86, 828-834
- **M. ALBENZIO et al.** Differential Leukocyte Count for Ewe Milk with Low and High Somatic Cell Count. *J. Dairy Research,* 2011, vol. 78, 43-48
- **I. COOPE.** Circle fitting by linear and nonlinear least squares. *Journal of Optimization Theory and Applications,* 1993, vol. 76 (2), 381
- **AKE BJORCK.** Numerical Methods for Least Squares Problems. *Society for Industrial and Applied Mathematics,* April 1996